# EUROPEAN PATENT APPLICATION

(11) **EP 3 827 770 A1**
(43) Date of publication of application: **02.06.2021**
(21) Application number: 18917753.8
(22) Date of filing: 06.11.2018
(51) Int. Cl.: A61B 17/34, A61B 90/50

(54) **ENDOSCOPE VIDEO CAMERA HEAD WHICH CAN BE ATTACHED TO A SURGICAL WOUND PROTECTOR, WITHOUT A RIGID TUBE OR MANUAL SUPPORT**

(30) Priority: 09.05.2018 ES 201830457; 13.07.2018 ES 201831118 U
(71) Applicant: Arroyo Tristan, Andres del Amor, 30170 El Niño de Mula (ES)
(72) Inventor: Arroyo Tristan, Andres del Amor, 30170 El Niño de Mula (ES)
(74) Representative: Munoz Garcia, Antonio
(86) International application number: PCT/ES2018/070714
(87) International publication number: WO 2019/215354

(57) **Abstract**

The invention relates to an endoscope video camera head which can be attached to a surgical wound protector without a rigid tube or manual support, comprising at least one video camera (3) with a light, focusing or zoom movements, means for power supply and connection for sending signals to a control unit and to one or more external screens. According to the invention, the head comprises a support structure (2) for the video camera (3) and means for power supply and connection for sending signals. The support structure is attached to a surgical wound protector (4) and is provided with coupling means (5) for attaching same to the surgical wound protector (4) in a temporary and removable manner or for attaching same to the protector (4) in a fixed and permanent manner or to the soft tissue wall (3), between the latter and the surgical wound protector (4), positioned on the external part of said protector.

## Description

### OBJECT OF THE INVENTION

As expressed in the title of the present specification, the invention relates to an endoscope video camera head which can be attached to a surgical wound protector without a rigid tube or manual support, providing the intended function thereof with advantages and features that are described in detail below and constitute a significant novelty in the current state of the art.

More specifically, the object of the invention focuses on an endoscope video camera head which is innovatively housed in a support along with means for supplying power to the camera, for supplying light, and for sending signals, forming a head which is particularly configured for the attachment thereof, in a temporary and removable manner or in a fixed and permanent manner, to a surgical wound protector of the types which are used in interventions consisting of endoscopic surgery, preferably abdominal, thoracic, or heart surgery, allowing, among other advantages, the possibility of visualizing body cavities eliminating the need for an assisting surgeon to be present in the surgical field to handle the camera head with a conventional rigid tube, as has been the norm so far. Furthermore, in an embodiment variant, the structure of the support is hollow and U-shaped, serving as an anchoring which includes said video camera incorporated therein or as an exostructure for the passage of a flexible endoscope therethrough.

### FIELD OF APPLICATION OF THE INVENTION

The field of application of the present invention is comprised within the health sector, specifically the sector of the industry dedicated to the manufacture of medical tools, apparatus, devices, and accessories, covering in particular the field of endoscope video cameras, and at the same time, the field of surgical wound protectors for minimally invasive surgery on body cavities.

### BACKGROUND OF THE INVENTION

As is known, endoscopy is a minimally invasive surgical procedure. It replaces large, body cavity access incisions with small incisions or ports through which endoscope video cameras with a rigid tube or endoscopic instruments are introduced for carrying out the same surgical procedures but in a less invasive manner, which results in less pain and a shorter postoperative stay for the patient. It is referred to by one name or another depending on the body cavity on which the procedure is performed, namely: abdomen-laparoscopy; thorax-thoracoscopy; etc.

In turn, the video camera system with a rigid tube comprises several components which are generally assembled in a movable metal structure referred to as a tower. It is made up of at least one display screen, a cold light unit, a camera control unit, a cable joining the camera control unit to the camera head, and a camera head joining the cable to the rigid tube.

Therefore, in the conventional visualization system consisting of a rigid tube, one end thereof is arranged outside the body cavity to be examined and is handled by an assisting surgeon through a handle, allowing it to be rotated 360° by hand. The central shaft of said rigid tube goes through the tissues to enter the body cavity to be examined. Additionally, the other tip end of the rigid tube, which is where the optics is located and from where the image is captured, enters the body cavity to be examined. This tip of the rigid tube can also have different angles.

Furthermore, there are other endoscopic instruments which are surgical instruments having a external handle handled by the surgeon, and a central shaft, going through the tissues to access the inside of the body cavity, and a tip with cutting, gripping, coagulation devices, etc.

Going back to the video camera, the signal captured by the optics at the tip of the rigid tube from the inside of the body cavity goes through the cable system of the tube to the handle and from said handle to the encoder and to the one or more screens. Said screens, the number and position of which depends on the positioning of the surgeons during surgery, make it possible for the lead surgeon and his or her assistants to visualize the surgery in real time.

Moreover, a surgical wound protector is a device which works as a retractor and surgical wound protector arising from the need to gain exposure and protection of the site of operation during an open surgical procedure or in endoscopic surgery ports. It is made up of a flexible internal ring, allowing access to the body cavity to be operated on, and another also flexible external ring, defining the inlet opening to said body cavity, and a likewise flexible, preferably plastic, tubular central body joining the rings between the internal and external parts of the different layers of skin or other tissues. Endoscopic instruments and/or the video camera with a rigid tube are introduced through this protector for carrying out the intervention.

Today, endoscopic surgery is performed with the patient lying down and with two or three scrubbed surgeons in the surgical field. An assisting surgeon usually handles the video camera with a rigid tube through a camera head existing at one of its ends, from where the signal exits by cable to the camera unit of the tower, and from said tower to the image-emitting monitors or screens. A lead surgeon who handles endoscopic instruments and one or more assisting surgeons assisting the lead surgeon to position the rest of the endoscopic instruments, are also present.

Therefore, the essential objective of the present invention focuses on the development of an innovative endoscope video camera head design which replaces the current camera design with a rigid tube that requires manual support and handling, taking advantage of the use of the described wound protectors in endoscopic interventions of this type for supporting same, and therefore allowing the need for the presence of the assisting surgeon dedicated exclusively to handling the camera to be prevented, which in turn entails a series of additional advantages.

Moreover, and as a reference to the current state of the art, it should be pointed out that the applicant at least is unaware of the existence of any other endoscope video camera head or any other invention with a similar application, having technical, structural, and constitutive features that are identical or similar to those of the camera head herein claimed.

### DESCRIPTION OF THE INVENTION

The endoscope video camera head which can be attached to a surgical wound protector without a rigid tube or manual support proposed by the invention is therefore configured as a novelty in its field of application, given that the objectives indicated above are specifically and satisfactorily achieved in view of its implementation, with the characterizing details making it possible and distinguishing same being suitably described in the final claims accompanying the present description.

More specifically, as set forth above, the invention proposes an endoscope video camera head which is innovatively configured as a support structure which, by housing at least one video camera with a light and zoom along with means for supplying power and sending signals and movement to direct the focus of the optics, has a configuration particularly designed for the attachment thereof, either in a temporary and removable manner or in a fixed and permanent manner, to a surgical wound protector of the types used in actual endoscopic surgery interventions in which said cameras are used, said head being devoid of the rigid tube incorporated in conventional heads existing today on the market.

Therefore, the essential objective of the present invention is to provide an endoscope video camera head which offers the following advantages while maintaining the quality and efficiency of the existing video camera systems:
- It eliminates the concept of endoscopy with a video camera having a rigid tube.
- It makes the presence of the assisting surgeon who handles the video camera in the surgical field unnecessary. The rotations and angles of the video camera requested by the lead surgeon during the surgical procedure to obtain a directed visualization of the anatomical structures to be dissected or removed can be ordered by an assisting surgeon or a nurse from the internal video camera from an external electronic device.
- It allows reducing the volume of apparatus required for the visualization of the intracavitary image, as well as the number of display screens required.
- It allows reducing the surgical wound instrumentation area in a single-port endoscopic surgery by at least one square centimeter (the square centimeter which the system with a rigid tube at least uses today). For example, in a single-port videothoracoscopic surgery in which a single incision measuring 2.5-3 cm is used, said free area offers a significant potential for instrumentation (more instruments or better angles).
- It offers an alternative to other inventions consisting of a single-port system (SILS) in which the contraposition of forces caused by the rigid tube of the optics on one hand, and the endoscopic instruments on the other, make it extremely difficult to achieve a comfortable intracavitary endoscopic surgery.
- In a single-port surgery with CO₂ insufflation, it is of vital importance so as not to interfere with or force instrumentation angles.

The head of the invention is furthermore susceptible to being used in combination with additional accessory elements such as, for example, a viewing system in individual glasses, sensors for detecting the movement of the iris of the lead surgeon for synchronizing the movement of the video camera according to the lead surgeon's intention on the display screen or viewing glasses, and it is also susceptible to being incorporated in a transportable carrying case to facilitate minimally invasive procedures in first-level hospitals or areas of humanitarian assistance (decentralization) without requiring bulky endoscope video systems.

To that end, the support structure forming the head of the invention which houses therein, as described, at least one video camera, means for supplying power and sending signals, and means for the angular movement and focusing of the optics, consists of, in a first embodiment variant, an elongated body which can be coupled in a removable manner to the inner wall of the surgical wound protector, specifically to the internal ring and the external ring of the protector such that these rings act as a support for the head, allowing the rotational movement thereof for positioning the camera which it houses incorporated at the lower end of said body.

In another embodiment variant, the support structure forming the head of the invention consists of a circular body housing one or more cameras which is coupled in a removable manner to the internal ring of the surgical wound protector, preferably anchored by pressure by means of a tongue-and-groove fitting of edges and recesses designed for such purpose in both parts, such that said ring act as a support for the head, allowing the axial rotation of said circular body on the mentioned internal ring.

In another alternative embodiment variant, the support structure forming the head of the invention, likewise consisting of a circular body housing one or more cameras, is attached to the surgical wound protector integrated therein, forming the actual internal ring of the protector, such that the surgical wound protector is at the same time the endoscope video camera head/heads.

In turn, the power supply of the camera and the cold light illuminating the inside of the body cavity reach same with several embodiment options that may include: both reaching by means of a cable from an external camera and cold light control unit to the inside of the body of the support of the head; both reaching without a cable by means of a battery which can be attached to the internal ring of the surgical wound protector and by means of wireless transmission communication with the control unit; or both reaching without cables such as by means of video cameras with a battery incorporated in the actual body of the head and by means of wireless transmission.

In summary, a first embodiment of the present invention intends to eliminate the endoscope video camera system based on a head with a rigid tube by replacing it with a head attached in the surgical wound protector which allows articulated mobility therein and keeps the endoscope port useful for surgical instrumentation. Furthermore, it eliminates the need for an assisting surgeon, a display screen for said assisting surgeon, and bulky visualization instruments. It intends to provide portability and efficiency to endoscopy.

Moreover, a second embodiment of the head of the invention proposes, where the purpose of the structure is to serve as a stable support for the use of at least one video camera for capturing images of the inside of body cavities in an endoscopic surgery, either as an anchoring including said video camera incorporated therein or as an exostructure for the passage of a flexible endoscope therethrough, also preventing the need to perform placement of the camera by means of a rigid tube handled by an assisting surgeon present in the surgical field, the mentioned support structure is designed with a U-shaped configuration allowing it to be used coupled, in the incision opening, to the tissue wall of the patient, along with the surgical wound protector, but on the external part of said protector and independently thereof, although without ruling out that it may be part of said protector, which provides the advantage of not taking up the space in the access opening to the internal body cavity defining the wound protector and of not requiring the adaptation or support thereof with respect to said protector, all this as a result, among other features, of the inside of said structure, which has a U-shaped configuration in this variant, being hollow and allowing the cables to reach the internal video camera from the outside for supplying power, for the video signal, for the cold light, and for controlling the camera rotation, movement, and zoom, when said camera is incorporated in a terminal of the structure itself, or allowing it to be configured as an exostructure for the passage of the flexible insertion tube of a flexible endoscope therethrough.

To that end, in this variant of the support structure with a U-shaped configuration, this structure essentially comprises an oblong and hollow generally U-shaped main body, determined by two parallel arms, one internal arm and another external arm, provided, respectively, for being positioned such that they are placed next to one another on the inner and outer parts of the tissue wall, and a perpendicular central arm joining said parallel arms to one another, being placed next to the tissue in the incision area, with an intracavitary terminal or an outlet port being at the distal end of the internal arm, through which the end of the flexible tube with the camera of the flexible endoscope emerges, and with a connection terminal or an inlet port of said tube being at the distal end of the external arm, depending on whether the variant is a structure for anchoring an integrated video camera or an exostructure for a flexible endoscope video camera.

Therefore, in one embodiment option, the support structure is provided as an anchoring for incorporating an integrated video camera, in which case, the intracavitary terminal of the distal end of the internal arm has a body housing the video camera and the light and is provided with the capacity for moving and zooming the camera, and the connection terminal of the distal end of the external arm is provided with a multiple connection plug socket body for connecting power supply, video signal, cold light, and for controlling the camera rotation, movement, and zoom, the wiring of which is prolonged through the inside of the described U-shaped body to the end with the intracavitary terminal.

Additionally, in an alternative embodiment option, the support structure of the invention is provided to serve as a tunnel track to a flexible endoscope for the internal visualization of body cavities. In this case, as a result of its hollow configuration, said structure (instead of wiring) allows serving as an exostructure and guide for the introduction of the flexible tube of a flexible endoscope for the visualization of body cavities.

It is important to point out that, in any case, said U-shaped body is made of a material imparting certain flexibility thereto for adaptation, by pressure, on both internal and external parts of the soft tissue wall in which it is incorporated, while still maintaining its U shape. For example, it is made of a plastic or metal material that is, in any case, compatible with the type of interventions for which it is intended.

Preferably, the U-shaped structure furthermore incorporates pressure-based holding means securing the anchoring thereof to the soft tissue wall, said means preferably being formed by a hand-tightened rotary knob which, when screwed, presses one of the parallel arms against the other.

In any case, said U-shaped body has a substantially planar cross-section configuration, such that it determines a minimum thickness on the soft tissue surface in the incision area, externally separating it from the wound protector, allowing the adaptation thereof to the tissue wall, which is particularly advantageous in the case of interventions requiring CO₂ insufflation.

Intracavitary CO₂ insufflation is usually performed through the surgical wound protector by means of an endoscope instrumentation hood coupled to said protector. Additionally, as it goes through the incision area, where it coincides with the flexible tubular body that is generally made of plastic and joins the internal and external rings of said wound protector, the planar shape of the structure creates an overlap between said flexible tubular body and the soft tissues, maintaining said inflation without leakage, as a result of the actual pressure of the insufflated CO₂.

Moreover, it should be pointed out that in the option of the structure designed as an anchoring with an integrated video camera, the body of the intracavitary terminal where the camera and the cold light are housed, in a first embodiment option, is a body with a ball-like spherical or semi-spherical configuration, having a rotational movement to enable orienting the camera in the appropriate angle during the course of the intervention. Additionally, in an alternative embodiment option, said body of the intracavitary terminal is a worm-like filiform body which is formed by a succession of rings joined to one another with articulated movement to allow orienting the camera as needed.

Lastly, it should be noted that the oblong and hollow main body with a U-shaped configuration of the structure optionally has a prolongation of the external arm, at the end of which there is incorporated the connection terminal or the inlet port of the flexible endoscope, extending defining an additional segment to facilitate access to said terminal, said prolongation preferably being oriented on the same plane as the mentioned external arm and preferably with an angle varying the orientation thereof, where said orientation can be varied depending on the needs of each case, for example, by means of the interposition of a flexible segment.

The described endoscope video camera head which can be attached to a surgical wound protector without a rigid tube or manual support represents an innovation in structural and constitutive features that have been unknown up until now, and these reasons, combined with its practical usefulness, provide it with sufficient grounds to obtain the exclusive privilege that is sought.

### DESCRIPTION OF THE DRAWINGS

To complement the description that is being made and for the purpose of helping to better understand the features of the invention, a set of drawings is attached to the present specification as an integral part thereof in which the following is depicted in a non-limiting, illustrative manner:

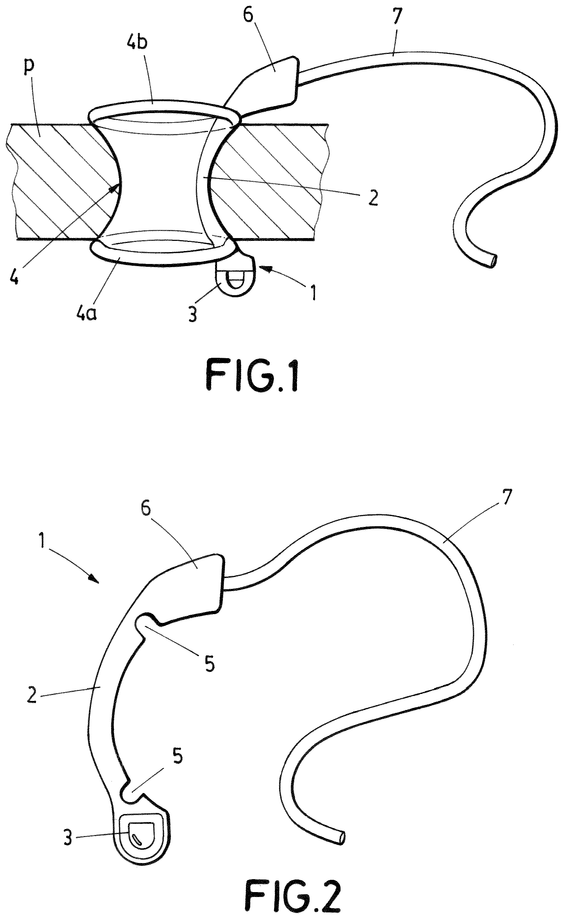

Figure 1 shows a schematic perspective view of an example of a first embodiment variant of the endoscope video camera head which can be attached to a surgical wound protector without a rigid tube or manual support, object of the invention, specifically a variant with a support structure which can be coupled, in a temporary and removable manner, to the inner wall of the surgical wound protector with rotational movement and with connection by means of a cable, said camera head has been depicted in a usage position coupled to said protector.
Figure 2 shows a perspective view of the example of the variant of the video camera head of the invention shown in Figure 1, in this case depicted without being coupled to the wound protector, where the main parts and elements it comprises, as well as the configuration and arrangement thereof, are shown.

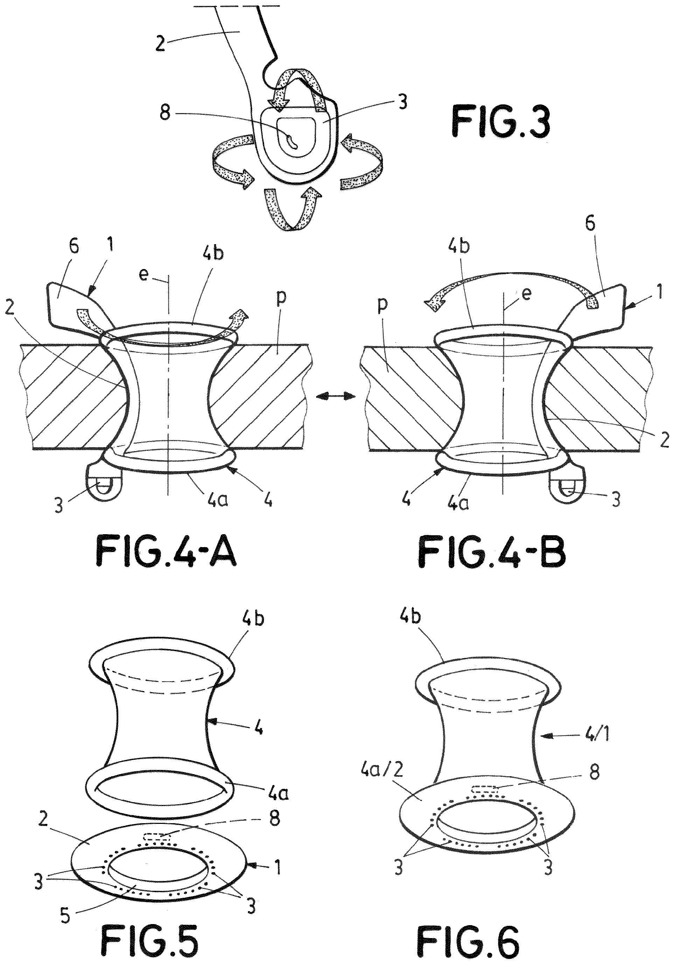

Figure 3 shows an enlargement of detail A indicated in Figure 2, showing the rotating end of the head in which the optics of the camera is incorporated.
Figures 4-A and 4-B show, in respective side perspective views, different usage positions the head is allowed to adopt, according to the embodiment variant shown in Figures 1 and 2.
Figure 5 shows a schematic perspective view of an example of another variant of the endoscope video camera head according to the invention, in this case a variant with a support structure which can be coupled, in a temporary and removable manner, to the lower ring of the surgical wound protector, depicted without being coupled to the head, and having in said example multiple cameras, powered with batteries and wireless connection.
Figure 6 shows a perspective view of an example of the head of the invention, with a shape very similar to that shown in Figure 5, but in a variant thereof which is joined in a fixed and permanent manner to the surgical wound protector, forming an integral part thereof.

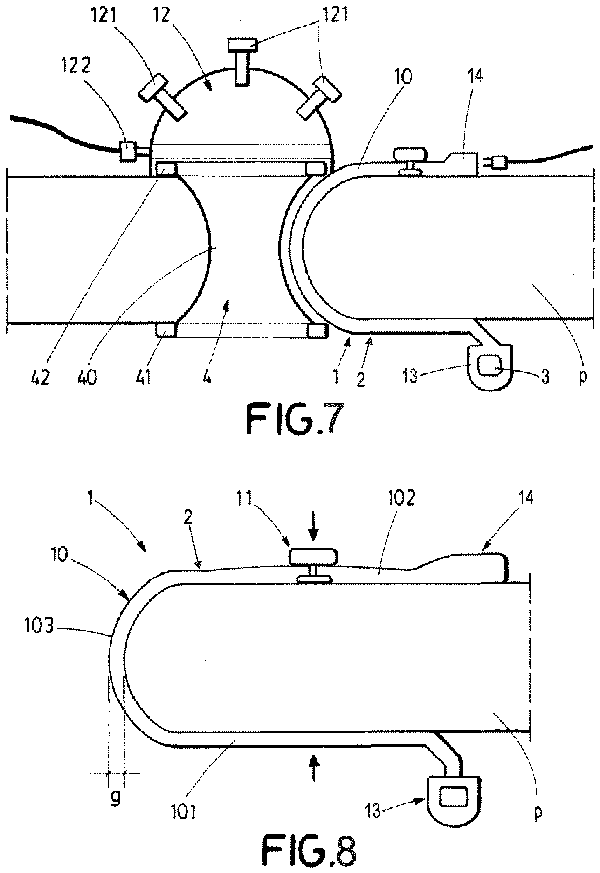

Figure 7 shows a schematic elevational view of an example of another variant of the head support structure object of the invention, specifically an example serving as an anchoring for the integrated camera, depicted once incorporated in the tissues of the patient along with the wound protector, being depicted in a section view and including means for insufflating CO₂, where the general configuration of said structure, the way in which it is incorporated outside the protector, and the arrangement of its main parts and elements inside and outside the body cavity, are shown.
Figure 8 shows a schematic elevational view of the head support structure according to the invention shown in Figure 7, in this case depicted in an independent manner, without the wound protector and coupled in the tissue wall shown in a section view, where the arrangement of the pressure-based fixing means it comprises for fixing to said tissues, are shown.

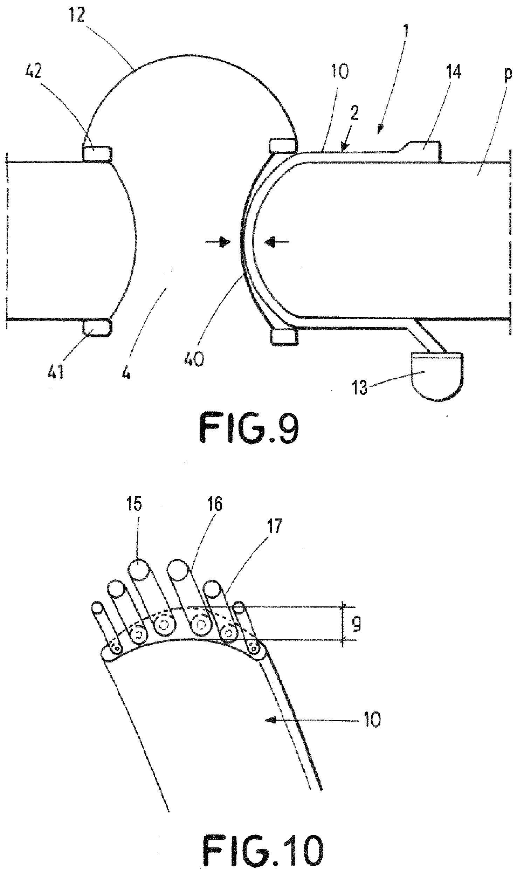

Figure 9 again shows a schematic elevational view of the structure of the head according to the example shown in Figures 7 and 8, in this case incorporated to the tissue wall, depicted in a section view, along with the wound protector, including the means for insufflating CO₂, where the attachment between the protector and the structure as a result of the actual pressure of the insufflated CO₂ is shown in this case.
Figure 10 shows a perspective view of a sectioned portion of the body of the structure of the head of the invention, where its hollow configuration for the passage of the wiring of the power supply, video signal, cold light connections, and for the control of camera rotation, movement, and zoom, are shown.

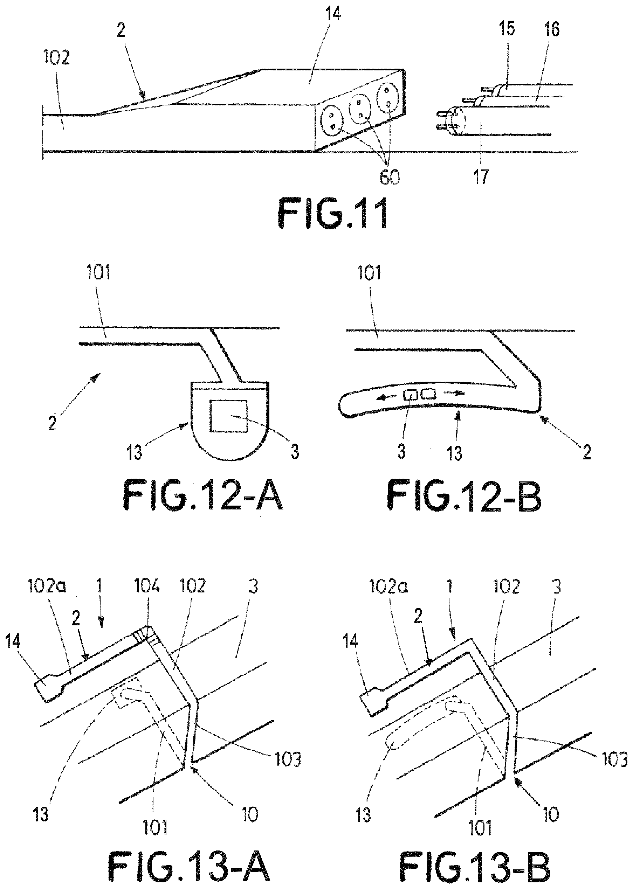

Figure 11 shows a schematic perspective view of the external terminal of the head support structure of the invention, where it incorporates the multiple connection plug socket body for supplying power, video signal, cold light, and for controlling the camera rotation, movement, and zoom.
Figures 12-A and 12-B show respective schematic perspective views of two different embodiment options of the internal terminal of the structure of the head according to the invention, where it incorporates the video camera, Figure 12-A showing an option in which said terminal is configured as a spherical body or in the shape of a ball, and Figure 12-B showing another option in which the internal terminal with the camera is configured as a filiform body or in the shape of a worm.
Figures 13-A and 13-B show respective schematic perspective views of the two options of the structure of the head of the invention shown in Figures 12-A and 12-B, in this case in a variant thereof with a perpendicular prolongation of the external arm of its U-shaped central body, where the arrangement of the parts thereof on both sides of the tissue wall, in which the structure is coupled, is shown in both cases.

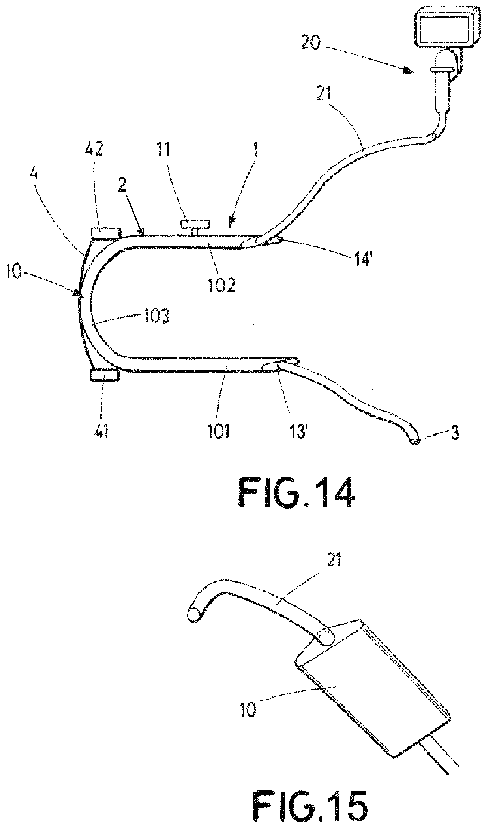

Figure 14 shows an elevational view of the structure of the head of the invention, similar to that shown in Figure 7, in this case of an example thereof in a variant as an exostructure for the passage of a flexible endoscope.
Figure 15 shows a perspective view of a portion of the hollow body of the structure, in a variant for a flexible endoscope, showing the passage of the flexible tube of the endoscope therethrough.

### PREFERRED EMBODIMENT OF THE INVENTION

In view of the mentioned figures and according to the numbering used, there can be seen therein several non-limiting embodiments of the endoscope video camera head which can be attached to a surgical wound protector of the invention, which comprises the parts and elements indicated and described in detail below.

Therefore, as can be seen in said figures, the head (1) at hand comprises a support structure (2) housing at least one video camera (3) with a light, focusing or zoom movements, means for power supply and connection for sending signals to a control unit and one or more external screens (not depicted), which support (2) is attached to a surgical wound protector (4) being joined thereto such that it is held in an independent manner and with the possibility of movement to orient the camera at will.

To that end, said configuration of the mentioned support (2) preferably comprises coupling means (5) for coupling to the surgical wound protector (4) in a temporary and removable manner (Figures 1 to 5) although, optionally, the configuration of the support (2) contemplates the attachment thereof to the protector (4) in a fixed and permanent manner, forming an integral part of the actual structure of the protector (4) (Figure 6).

Therefore, in a first embodiment variant, like the one shown in Figures 1 to 4, the support (2) of the head (1) consists of a hollow and elongated body which can be coupled, in a removable manner, to the inner wall of the surgical wound protector (4), specifically to the internal ring (4a) and the external ring (4b) of the protector by means of respective slots provided as the coupling means (5), allowing the rotational movement thereof about an axial axis (e) of the protector (4) for positioning the camera (3) which it houses incorporated at the lower end of said body of the support (2) in the position of interest.

Preferably, to handle the head (1) and carry out said rotation, the body of the support (2) has an upper prolongation defining a handling flange (6).

In this variant, the power supply and connection for sending signals to the control unit and one or more external screens are preferably done through cables (7) inserted in the support (2), without this entailing a limitation, since they can also be done through batteries (8) and a wireless communication module that can be housed inside the support (2) or connected outside same fixed in the surgical wound protector (4).

Figure 1 and Figures 4-A and 4-B depict this variant of the head (1) with the elongated body of the support (2) incorporated in the protector (4) fixed in the working position, i.e., with its respective internal ring (4a) and external ring (4b) supported in both internal and external parts of the skin or soft tissue wall (p).

Furthermore, as can be seen in Figure 3, in this embodiment variant the video camera (3) positioned at the lower end of the elongated body of the support (2) has a battery (8) and presents rotational movement.

In another embodiment variant like the one shown in Figure 5, the support (2) forming the head (1) is made up of a hollow body with a circular configuration which is coupled, in a removable manner, on the internal ring (4a) of the surgical wound protector (4), for example by pressure, by means of a tongue-and-groove fitting of edges and recesses provided as coupling means (5) in both parts, allowing the axial rotation of said circular body of the support (2) on the mentioned internal ring (4a).

In this variant, the circular body of the support (2) preferably houses several cameras (3) radially distributed therein. Additionally, the power supply and connection for sending signals to the control unit and external screens are also preferably done through batteries (8) and wireless communication.

In another alternative embodiment variant like the one shown in Figure 6 where the support (2) of the head (1) is also a circular body housing one or more cameras (3) similar to the one shown in Figure 5, said support constitutes the actual internal ring (4a) of said protector (4), forming an integral part thereof, instead of being coupled to the internal ring (4a) of the surgical wound protector (4).

In this variant, the support (2) also preferably houses several cameras (3) radially distributed therein and the power supply and connection for sending signals to the control unit and screens external are done through batteries (8) and wireless communication, where it must be understood that both in this variant and in the one shown in Figure 5, the inclusion of batteries (8) and wireless communication does not constitute a limitation because, though not depicted, it could also be connected by means of a cable (7).

In reference to Figures 7 to 15, another embodiment variant of the head (1) of the invention can be seen, where the support structure (2), which is applicable as a support for the use of at least one video camera (3) with a light and can be inserted in the internal body cavity object of an endoscopic intervention, with focusing or zoom movements, connected through means for power supply and connection for controlling and sending signals to a unit with one or more external screens or incorporated in a flexible endoscope (20), is hollow and has a general U-shaped configuration, suitable for the coupling thereof, in the incision opening, to the soft tissue wall (p), between the latter and the surgical wound protector (4) incorporated in said incision, positioned on the external part of said protector, again preferably as an element independent of said protector, although without ruling out that it may be part of said protector, in any case, comprising an intracavitary terminal (13) for the integrated video camera (3) or an outlet port (13') for the camera (3) of a flexible endoscope (20), and an external connection terminal (14) for supplying power, video signal, cold light, and for controlling said integrated camera (3), or an inlet port (14') for said flexible endoscope (20).

To that end, the support structure (2) preferably comprises an oblong and hollow U-shaped main body (10), determined by two parallel arms, one internal arm (101) and another external arm (102), provided, respectively, for being positioned such that they are placed as closely as possible next to one another on the inner and outer parts of the soft tissue wall (p), and a central arm (103) perpendicular to said arms joining them together, being placed next to the soft tissue (p) between the latter and the flexible tubular body (40) joining the internal ring (4a) and the external ring (4b) of the wound protector (4) covering the incision opening, with the intracavitary terminal (13) or the outlet port (13') being at the distal end of the internal arm (101) and a connection terminal (14) or the inlet port (14') at the distal end of the external arm (102), depending on whether the variant is a structure for anchoring an integrated video camera (3) or an exostructure for a flexible endoscope video camera (20).

When the support structure (2) serves as an anchoring for an integrated video camera (3), Figures 7 to 13, the intracavitary terminal (13) of the distal end of the internal arm (101) is formed by a body housing the video camera (3) and the light and is provided with the capacity for moving and zooming the camera, and the connection terminal (14) of the distal end of the external arm (102) is preferably formed by a multiple connection female plug socket (60) for connecting at least the power supply connector (15) for controlling the camera movement and zoom, the video signal connector (16), and the cold light connector (17), as shown in Figure 11, and the wiring of which is prolonged through the hollow interior of said U-shaped main body (10) from said connection terminal (14) to the opposite end of the intracavitary terminal (13).

Additionally, when the structure (2) is used for a flexible endoscope (20), the flexible tube (21) thereof goes through the hollow body (10) of the structure, inserted through the inlet port (14') of the external arm (102) and emerging with the camera (3) through the outlet port (13') on the opposite side in the internal arm (101), as can be seen in Figures 14 and 15.

At least the described U-shaped main body (10) of the structure (2) is preferably made of a slightly flexible material to allow the separation of the parallel arms (101, 102) for carrying out the placement thereof, surrounding the soft tissue (p) internally and externally, and with the capacity to recover its U shape once being placed.

In any case, the structure (2) also preferably comprises pressure-based holding means (11) securing the anchoring thereof to the soft tissue wall (p), consisting of at least one hand-tightened rotary knob located, as shown in Figure 8, in the external arm (102) of the structure, for example.

In any case, the U-shaped main body (10) of the structure, as can be seen in Figure 10, has a planar cross-section configuration determining a thickness (g) of a small dimension, necessary for the passage of the power supply cable (15) for controlling the camera movement and zoom, the video signal cable (16), and the cold light cable (17), or for the passage of the flexible tube (21) of the flexible endoscope (20), such that it projects minimally above the soft tissue surface (p) between the latter and the flexible tubular body (40) of the wound protector (4), allowing, in intracavitary CO₂ insufflation operations performed by means of an endoscope instrumentation hood (12) coupled to the wound protector (4), the creation an overlap between said flexible tubular body (40) and the soft tissue wall (p), maintaining said inflation without leakage, as a result of the actual pressure of the insufflated CO₂, as shown by the arrows of Figure 9.

Figure 7 depicts the structure (2) of the head of the invention along with the wound protector (4) provided with the mentioned endoscope instrumentation hood (12) in which, in addition to various connection points (121), the CO₂ insufflation connection (122) has been depicted.

In reference to Figure 12-A it can be seen how, when the structure (2) serves as an anchoring for an integrated video camera (3) in an embodiment option, the intracavitary terminal (13) where the video camera (3) with the cold light is housed is a body with a ball-like spherical or semi-spherical configuration, having a rotational movement to enable orienting said video camera (3) as needed. Additionally, Figure 12-B shows another embodiment option in which the intracavitary terminal (13) is a filiform body with articulated movement.

Additionally, Figures 13-A and 13-B show how, in both options of the described spherical or filiform configurations of the intracavitary terminal (13) with the video camera (3), the U-shaped main body (10) optionally has a prolongation (102a) of its external arm (102), at the distal end of which it incorporates the connection terminal (14), defining an additional segment preferably extending on the same plane as said external arm (102) and at the same or different angle of orientation, and optionally having a flexible segment (104), for example a bellows-like segment, interposed therein, so as to enable varying said plane and/or said angle of orientation, as shown in Figure 13-A. It should be pointed out that said prolongation (102a) is also suitable for the variant of the structure (2) when it is used for the passage of a flexible endoscope (20).

Having sufficiently described the nature of the present invention, as well as the manner of putting it into practice, it is not considered necessary to expand on this explanation so that one skilled in the art will understand its scope and the advantages derived from it, hereby stating that within its essential nature, the invention may be carried out to practice in other embodiments which differ in detail from that indicated by way of example and which will likewise attain the protection that is sought provided that its fundamental principle is not altered, changed, or modified.

## Claims

1. An endoscope video camera head which can be attached to a surgical wound protector without a rigid tube or manual support, comprising at least one video camera (3) with a light, focusing or zoom movements, means for power supply and connection for sending signals to a control unit and to one or more external screens, **characterized by** comprising a support structure (2) for the at least one video camera (3), means for power supply and connection for sending signals, which support (2) is attached to a surgical wound protector (4), being joined thereto such that it is held in an independent manner and with the possibility of movement to orient the camera at will.

2. The endoscope video camera head which can be attached to a surgical wound protector without a rigid tube or manual support according to claim 1, **characterized in that** the support (2) is provided with coupling means (5) for attaching same to the surgical wound protector (4) in a temporary and removable manner.

3. The endoscope video camera head which can be attached to a surgical wound protector without a rigid tube or manual support according to claim 2, **characterized in that** the support (2) consists of a hollow and elongated body which can be coupled, in a removable manner, to the inner wall of the surgical wound protector (4), with rotational movement about an axial axis (e) of the protector (4) for positioning the camera (3) which it houses incorporated at the lower end of said body of the support (2) in the position of interest.

4. The endoscope video camera head which can be attached to a surgical wound protector without a rigid tube or manual support according to claim 3, **characterized in that** the body of the support (2) has respective slots as coupling means (5) for coupling to the internal ring (4a) and the external ring (4b) of the protector (4).

5. The endoscope video camera head which can be attached to a surgical wound protector without a rigid tube or manual support according to claim 3 or 4, **characterized in that** the body of the support (2) has an upper prolongation defining a handling flange (6) for causing it to rotate about the protector (4).

6. The endoscope video camera head which can be attached to a surgical wound protector without a rigid tube or manual support according to claim 2, **characterized in that** the support (2) is made up of a hollow body with a circular configuration housing one or more cameras (3) radially distributed therein and is coupled on the internal ring (4a) of the surgical wound protector (4), allowing the axial rotation of said circular body of the support (2) on the mentioned internal ring (4a).

7. The endoscope video camera head which can be attached to a surgical wound protector without a rigid tube or manual support according to claim 1, **characterized in that** the support (2) is attached to the protector (4) in a fixed and permanent manner, forming an integral part of the actual structure thereof.

8. The endoscope video camera head which can be attached to a surgical wound protector without a rigid tube or manual support according to claim 7, **characterized in that** the support (2) is made up of a hollow body with a circular configuration housing one or more cameras (3) radially distributed therein and making up the internal ring (4a) itself of the surgical wound protector (4), forming an integral part thereof.

9. The endoscope video camera head which can be attached to a surgical wound protector without a rigid tube or manual support according to any of claims 1 to 8, **characterized in that** the support (2) incorporates cables inserted therein for power supply and connection for sending signals to the control unit and one or more external screens.

10. The endoscope video camera head which can be attached to a surgical wound protector without a rigid tube or manual support according to any of claims 1 to 8, **characterized in that** the support (2) incorporates batteries (8) and a wireless communication module which are housed therein for power supply and connection for sending signals to the control unit and one or more external screens.

11. The endoscope video camera head which can be attached to a surgical wound protector without a rigid tube or manual support according to any of claims 1 to 8, **characterized in that** the support (2) incorporates batteries (8) and a wireless communication module which are housed outside same, fixed in the surgical wound protector (4) for power supply and connection for sending signals to the control unit and one or more external screens.

12. The endoscope video camera head which can be attached to a surgical wound protector without a rigid tube or manual support according to claim 1, **characterized in that** the structure configuring the support (2) is hollow and has a general U-shaped configuration, suitable for the coupling thereof, in the incision opening, to the soft tissue wall (p), between the latter and the surgical wound protector (4) incorporated in said incision, positioned on the external part of said protector, and comprising an integrated intracavitary terminal (13) for the video camera (3) or an outlet port (13') for the camera (3) of a flexible endoscope (20), and an integrated external connection terminal (14) for supplying power, video signal, cold light, and for controlling said camera (3), or an inlet port (14') for said flexible endoscope (20).

13. The endoscope video camera head which can be attached to a surgical wound protector without a rigid tube or manual support according to claim 12, **characterized in that** the support structure (2) with a U-shaped configuration is an element independent of the wound protector (4).

14. The endoscope video camera head which can be attached to a surgical wound protector without a rigid tube or manual support according to claim 13, **characterized in that** the support structure (2), when it serves as an anchoring for the integrated video camera (3), comprises an oblong and hollow U-shaped main body (10), determined by two parallel arms, one internal arm (101) and another external arm (102), provided, respectively, for being positioned such that they are placed as closely as possible next to one another on the inner and outer parts of the soft tissue wall (p), and a central arm (103) perpendicular to said arms joining them together being placed next to the soft tissue (p) between the latter and the flexible tubular body (40) joining the internal ring (4a) and the external ring (4b) of the wound protector (4) covering the incision opening, with the intracavitary terminal (13) being at the distal end of the internal arm (101) and a connection terminal (14) at the distal end of the external arm (102).

15. The endoscope video camera head which can be attached to a surgical wound protector without a rigid tube or manual support according to claim 14, **characterized in that** the connection terminal (14) is formed by a multiple connection female plug socket (60) for connecting at least the power supply connector (15) for controlling the movement and zoom of the camera, the video signal connector (16), and the cold light connector (17), the wiring of which is prolonged through the hollow interior of the U-shaped main body (10) from said connection terminal (14) to the opposite end of the head (1).

16. The endoscope video camera head which can be attached to a surgical wound protector without a rigid tube or manual support according to claim 14 or 15, **characterized in that** the intracavitary terminal (13) with the video camera (3) and the cold light is a body with a ball-like spherical or semi-spherical configuration, having a rotational movement to enable orienting said video camera (3) as needed.

17. The endoscope video camera head which can be attached to a surgical wound protector without a rigid tube or manual support according to claim 14 or 15, **characterized in that** the intracavitary terminal (13) with the video camera (3) and the cold light is a body having a filiform configuration with articulated movement.

18. The endoscope video camera head which can be attached to a surgical wound protector without a rigid tube or manual support according to claim 12, **characterized in that** the support structure (2) comprises an oblong and hollow U-shaped main body (10), determined by two parallel arms, one internal arm (101) and another external arm (102), provided, respectively, for being positioned such that they are placed as closely as possible next to one another on the inner and outer parts of the soft tissue wall (p), and a central arm (103) perpendicular to said arms joining them together, being placed next to the soft tissue (p) between the latter and the flexible tubular body (40) joining the internal ring (4a) and the external ring (4b) of the wound protector (4) covering the incision opening, with the outlet port (13') being at the distal end of the internal arm (101) and the inlet port (14') being at the distal end of the external arm (102).

19. The endoscope video camera head which can be attached to a surgical wound protector without a rigid tube or manual support according to any of claims 12 to 18, **characterized in that** the support structure (2) also comprises pressure-based holding means (11) securing the anchoring thereof to the soft tissue wall (p).

20. The endoscope video camera head which can be attached to a surgical wound protector without a rigid tube or manual support according to claim 19, **characterized in that** the pressure-based holding means (11) consist of at least one hand-tightened rotary knob located in the external arm (102) of the support structure (2).

21. The endoscope video camera head which can be attached to a surgical wound protector without a rigid tube or manual support according to any of claims 12 to 20, **characterized in that** the U-shaped main body (10) has a planar cross-section configuration determining a thickness (g) of a small dimension projecting minimally above the soft tissue surface (p) between the latter and the flexible tubular body (40) of the wound protector (4), creating in intracavitary CO₂ insufflation operations, as a result of the actual pressure of the insufflated CO₂, an overlap between said flexible tubular body (40) and the soft tissue wall (p) which maintains said inflation without leakage.

22. The endoscope video camera head which can be attached to a surgical wound protector without a rigid tube or manual support according to any of claims 12 to 21, **characterized in that** the U-shaped main body (10) has a prolongation (102a) of its external arm (102), at the distal end of which it incorporates the connection terminal (14) or the inlet port (14'), defining an additional segment.

23. The endoscope video camera head which can be attached to a surgical wound protector without a rigid tube or manual support according to claim 22, **characterized in that** the prolongation (102a) of the external arm (102) extends on the same plane as said arm, with the same or different angle of orientation.

24. The endoscope video camera head which can be attached to a surgical wound protector without a rigid tube or manual support according to claim 23, **characterized in that** a flexible segment (104) is interposed in the prolongation (102a) of the external arm (102) to enable varying the plane and/or the angle of orientation thereof.

25. The endoscope video camera head which can be attached to a surgical wound protector without a rigid tube or manual support according to any of claims 12 to 24, **characterized in that** at least the U-shaped main body (10) of the support structure (2) is made of a slightly flexible material with the capacity to recover its U shape.
